# EUROPEAN PATENT SPECIFICATION

(11) **EP 3 887 047 B1**
(45) Date of publication and mention of the grant of the patent: **10.06.2026**
(21) Application number: 19816422.0
(22) Date of filing: 28.11.2019
(51) Int. Cl.: B01L 3/00, B01L 7/00

(54) **IMPROVED MICROFLUIDIC DEVICES, SYSTEMS AND METHODS**
VERBESSERTE MIKROFLUIDIKVORRICHTUNGEN, SYSTEME UND VERFAHREN
DISPOSITIFS, SYSTÈMES ET PROCÉDÉS MICROFLUIDIQUES AMÉLIORÉS

(30) Priority: 29.11.2018 GB 201819419
(43) Date of publication of application: 06.10.2021
(73) Proprietor: QuantuMDx Group Limited, Newcastle upon Tyne, Tyne and Wear NE1 2JQ (GB)
(72) Inventor: MADADI, Hojjat, Newcastle Upon Tyne Tyne and Wear NE1 2JQ (GB); WILLSHARE, Thomas Michael, Newcastle Upon Tyne Tyne and Wear NE1 2JQ (GB); PAGE, Robin, Newcastle Upon Tyne Tyne and Wear NE1 2JQ (GB)
(74) Representative: Definition IP Limited
(86) International application number: PCT/GB2019/053367
(87) International publication number: WO 2020/109801

(56) References cited:
- US-A1- 2009 148 933
- US-A1- 2010 129 872
- US-A1- 2011 039 303
- US-A1- 2018 311 673

## Description

### Technical Field

The present invention relates to microfluidic devices, such as cassettes, for diagnostics, which allow for on-cassette processing including amplification by polymerase chain reaction (PCR), and systems and methods of using the same, wherein microfluidic cassette is pressurised prior to amplification of nucleic acid in said sample in an amplification/PCR section of the device.

### Background

In recent years there have been ongoing efforts to develop and manufacture microfluidic devices, such as cassettes, that are able to perform various chemical and biochemical analyses and syntheses on device. The goal is to provide devices that allow previously laboratory-based activities to be performed at the point of care (POC) in a timely and efficient manner. Such microfluidic devices can be adapted for use with automated systems, thereby providing the additional benefits such as cost reductions and decreased operator errors.

In many cases it is desirable that such microfluidic devices are capable of carrying out molecular techniques including the amplification of nucleic acids. Amplification of DNA by polymerase chain reaction (PCR) requires reaction mixtures be subjected to repeated rounds of heating and cooling (thermocycling) whist travelling through microfluidic channels present on the cassette, which can include holding the reaction mixtures in one or more static chambers present on the cassette. The temperature of the reaction mixture within the cassette therefore must be varied during a PCR cycle, and in fact varied many times during a PCR experiment, which typically requires a relatively high number of cycles to obtain a suitable amplification of the nucleic acid from the sample. The variation of the temperature within the cassette does bring technical challenges. For example, denaturation of DNA typically takes place at greater than 90 degrees, and often close to 98 degrees C. The temperature then needs to drop as annealing a primer to the denatured DNA is typically performed at around 45 degrees to 70 degrees C. Finally, the temperature is raised again as the step of extending the annealed primers with a polymerase is typically performed at around 70 degrees to 75 degrees C. Various mechanisms to allow for this have been described, with systems employing heated zones being utilised, or means for rapidly changing the temperature within microfluidic channels being employed. Such devices and systems are known.

One challenge associated with the rapid cycling of temperatures is that, particularly at the higher temperatures, bubbles coming out of the sample fluid can decrease the PCR efficiency and also make accurate positioning of the sample less predictable. US2009/148933 A1 discloses a microfluidic device comprising a microfluidic channel having a polymerase chain rection (PCR) section, a fluidically isolatable portion of the device comprising at least the portion of the fluidic channel having a PCR section, at least one closure means actuatable to fluidically isolate the fluidically isolatable portion from the external environment.

It would be beneficial to obviate or mitigate some of the problems associated with the prior art.

The term 'Shuttle flow' or 'Shuttle PCR' refers to techniques in which thermocycling is performed by shuttling small plugs of PCR mixture back and forth between temperature zones. In this way temperature variations occur spatially.

Throughout this document reference to "microfluidic" means with at least one dimension less than 1 millimetre and/or able to deal with microlitre or less portions of fluid.

Throughout this document reference to "cassette" or "chip" means an assembled unit comprising one or more substrates with channels or chambers therein through which fluid can flow. Such cassettes may include different regions or zones in which activities such as sample mixing, filtering, PCR amplification, identification and/or visualisation can occur and may include on-board reagents. The cassettes are typically designed to be received by a diagnostic instrument such as a point-of-care (POC) instrument which incorporates additional functionality to allow a diagnostic test, or part of such a test, to be automated.

### Summary of the Invention

According to a first aspect of the present invention there is provided a microfluidic device comprising;
a microfluidic channel having a polymerase chain reaction (PCR) section, said PCR section configured to allow the temperature changes required for polymerase chain reaction (PCR) to occur therein,
a fluidically isolatable portion of the device (or portion of the fluidic channel) comprising at least the portion of the fluidic channel having a PCR section,
at least one closure means actuatable to fluidically isolate the fluidically isolatable portion from the external environment, and
a first bellows pump and a second bellows pump, and; wherein, the first bellows pump and the second bellows pump are arranged such that in use they are simultaneously or concurrently compressed by a first amount to a first position to increase the pressure in the isolatable portion to a raised pressure, and then the first pump is further compressed by a second amount and the second pump allowed to reinflate to move a liquid sample within the channel without further increasing the pressure in the isolatable portion beyond the raised pressure.

A PCR section of the channel is a portion of the channel that has been configured to allow thermocycling of a sample passing therethrough to occur. The PCR section of the channel is specifically adapted to allow a liquid sample travelling therethrough to be heated and cooled in a cyclical manner to temperatures that allow for denaturing of DNA, annealing of DNA and amplification of DNA. The PCR section of the channel is heatable either by an integral heat source within the channel or the wall of the device, or more typically by being brought into the proximity of an external heat source which applies heat to microfluidic device (such as a microfluidic cassette) in a manner that allows heat to transfer into the PCR section of the channel.

Importantly, the first and second bellows pumps are arranged such that in use they are simultaneously or concurrently compressed by a first amount to a first position to increase the pressure in the isolatable portion to a raised pressure, and are adapted to increase pressure within the PCR section of the channel prior to amplification of a sample by thermocycling within the PCR section.

The microfluidic device is suitable for receiving a liquid sample. Preferably it is a microfluidic cassette for insertion into a point of care (POC) diagnostic instrument.

The microfluidic channel is for transporting said liquid sample and has an inlet end and at least one second end that are in fluid communication with each other.

Most preferably the circumference of the channel is fluid-tight.

Throughout this document, the term 'fluid-tight' means a sealing that prevents the passage of liquids, such as water, and/or gases, in particular air, even if put under pressure (within predetermined limits). For example, the seal will remain intact up to approximately 200kPa (2 bar) of pressure difference between the interior and exterior sides of the slide fastener.

Optionally, at least part of the microchannel has a serpentine configuration.

Advantageously serpentine configurations can allow long fluid flow in a relatively small footprint, which means an efficient heat transfer by increasing surface to volume ratio.

In some embodiments the serpentine configuration can be useful in allowing fluid to travel over multiple different temperature zones, for example different heat plates, before curving back to repeat the process. In other embodiments the serpentine configuration can be utilised along with methods of shuttling fluid back and forth between temperature zones (i.e. shuttle flow (shuttle PCR) or

The isolatable portion may be the entire fluidic channel present on the device. In one embodiment, substantially all of the microfluidic channel present on a cassette is the isolatable portion. In this embodiment, this means that once a sample is inserted into the cassette i.e. inserted into the start of the microfluidic channel, the channel is then sealed, and the pressure is increased.

Advantageously, using positive pressure (i.e. inducing a positive pressure within the a microfluidic channel prior to significant further activity) in other microfluidic channels within a device such as a cassette, as well as in the PCR section helps to minimise fluid(liquid)-plug breakup i.e. break-up of the sample fluid plug rather than maintaining it as a single plug of fluid (the sample plug may incorporate additional reagents as it moves through the cassette). Having positive pressure acts to keep the meniscus 'tight' and prevent fluid 'creep' along the walls which can then coalesce at point up/down stream of the main plug, creating two-plugs with an intervening air/gas bubble (this is a particular issue in the PCR section as the intervening air/gas bubble, which is not generated through de-gassing, can occur in the PCR region owing to the higher temperatures that the sample plug is subjected to.

Preferably the at least one closure means is at least one air-tight valve or sealing means actuatable to fluidically isolate the fluidically isolatable portion.

The bellows pump is a substantially hemispherical compressible material with an internal cavity. The bellows pump is associated with an inlet to the microfluidic channel. When pressure is applied to the external surface of the bellows pump the material is compressed such that the internal cavity is reduced in volume and any fluid in the internal cavity is pushed.

The bellows pump is resiliently biased to return to an expanded position.

An external pressure source e.g. mechanical, pneumatic or hydraulic source of pressure can be applied by an external host instrument.

Advantageously by simultaneously increasing the pressure in the portion of the microchannel including the amplification zone using multiple means to increase pressure e.g. two or more positive displacement pumps, this allows the pressure in the portion of the microchannel that includes the amplification zone to be increased and then the means to increase pressure can be further used to move liquid sample within the microchannel without placing a further pressure load on the system.

Preferably the means to increase the pressure are actuable to a first compression position and a second compression position.

More preferably the second compression position is further compressed (thus having less volume within it) than the first position.

The PCR section is heatable.

An external source of heat can be applied by an external instrument.

Preferably the means for increasing pressure in the isolatable portion induces a pressure higher than atmospheric pressure.

Preferably the means for increasing pressure in the isolatable portion induce a pressure of 160kPa (1.6bar) or higher.

Optionally the means for increasing pressure in the isolatable portion induce a pressure of 120kPa (1.2bar) or higher.

Preferably the microfluidic device comprises on-board reagents for performing at least one diagnostic assay.

According to another aspect of the present invention there is provided a diagnostic system comprising the microfluidic device of the first aspect, and a host instrument able to receive said microfluidic device.

Optionally, the host instrument comprises an interface enabled to convey pressure from said host instrument to said means for increasing pressure on said microfluidic device.

Optionally the host instrument comprises a pneumatic interface enabled to convey pneumatic pressure from said host instrument to said means for increasing pressure on said microfluidic device.

Optionally, the host instrument comprises a mechanical interface enabled to convey mechanical pressure from said host instrument to said means for increasing pressure on said microfluidic device.

Preferably the host instrument is adapted to actuate an increased pressure in the isolatable portion of the cassette when it is isolated and prior to amplification of a sample by thermocycling within the PCR section.

Preferably the host instrument comprises a microprocessor.

The microprocessor controls the interactions between the host instrument to the microfluidic device.

Optionally the host instrument comprises means for heating or applying heat to at least a portion of the PCR section of the device.

Preferably the means for heating is one or more temperature-controlling elements configured to provide at least one temperature zone.

According to a further aspect of the present invention there is a method of amplifying nucleic acid from a sample comprising, providing the device or system of the above aspects,
actuating the at least one closure means and fluidically isolating at least a portion of the device, more particularly the portion of the microfluidic channel comprising at least the PCR section,
increasing the pressure within the isolated portion,
and, after the pressure has been increased within the isolated portion, carrying out polymerase chain reaction (PCR) amplification steps within the isolated portion to amplify nucleic acid within the sample.

Preferably the sample is inserted into the device prior to the step of fluidically isolating at least a portion of the device comprising at least the PCR section.

Optionally the entire device is fluidically isolated.

By providing a device with isolatable portions that are fluid-tight, and particularly air-tight, the cassette can be pre-pressurised prior to polymerase chain reaction (PCR) thermocycling occurring.

Alternatively, only portions of the microfluidic channel are fluidically isolated.

By isolating only portions of the microfluidic channel containing the PCR zone, other branches of the channel can be closed off, including branches to reservoirs etc, either to prevent ingress of materials detrimental to PCR reactions or to reduce the volume to which pressure is applied.

Optionally one or more fluid-tight valves are used to fluidically isolate the system.

The valves may be within the channels or form the inlet (or outlet if present).

Making the device such as a microfluidic cassette, or a portion thereof, air-tight allows pressure to then be increased.

Advantageously by using a first and a second bellows pumps, this allows the pressure in the portion of the microchannel that includes the amplification zone to be increased without leakage.

Optionally the step of increasing the pressure increases the pressure in the isolated portion to greater than 120 kPa (1.2bar) , more preferably to greater than 140 kPa (1.4bar) yet more preferably greater than 160 kPa (1.6bar).

Optionally the step of increasing the pressure increases the pressure in the isolated portion to between 150 kPa (1.5bar) and 200 kPa (2bar).

Preferably the step of increasing the pressure increases the pressure in the isolated portion to 160 kPa (1.6bar).

Throughout this document reference to "microfluidic" means with at least one dimension less than 1 millimetre and/or able to deal with microlitre or less portions of fluid.

Various further features and aspects of the invention are defined in the claims.

### Brief Description of the Drawings

Embodiments of the present invention will now be described by way of example only with reference to the accompanying drawings in which like parts are provided with corresponding reference numerals and in which:

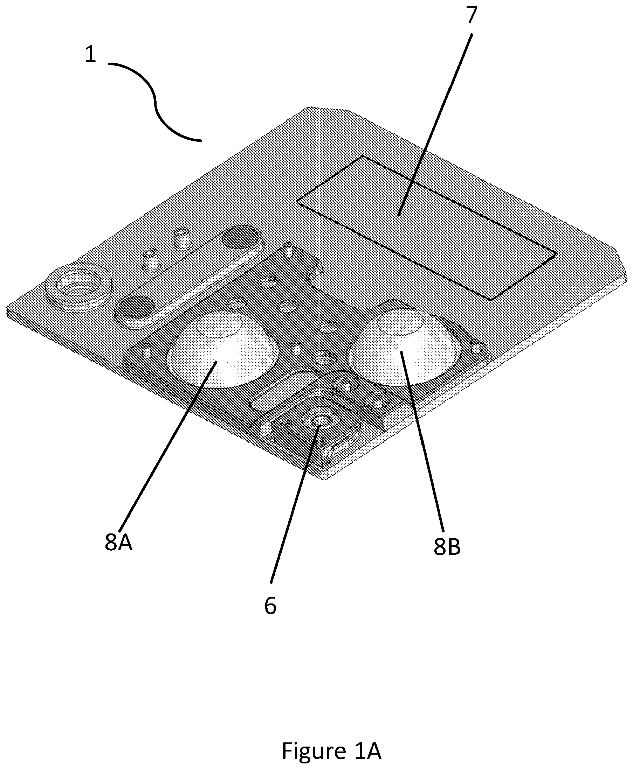

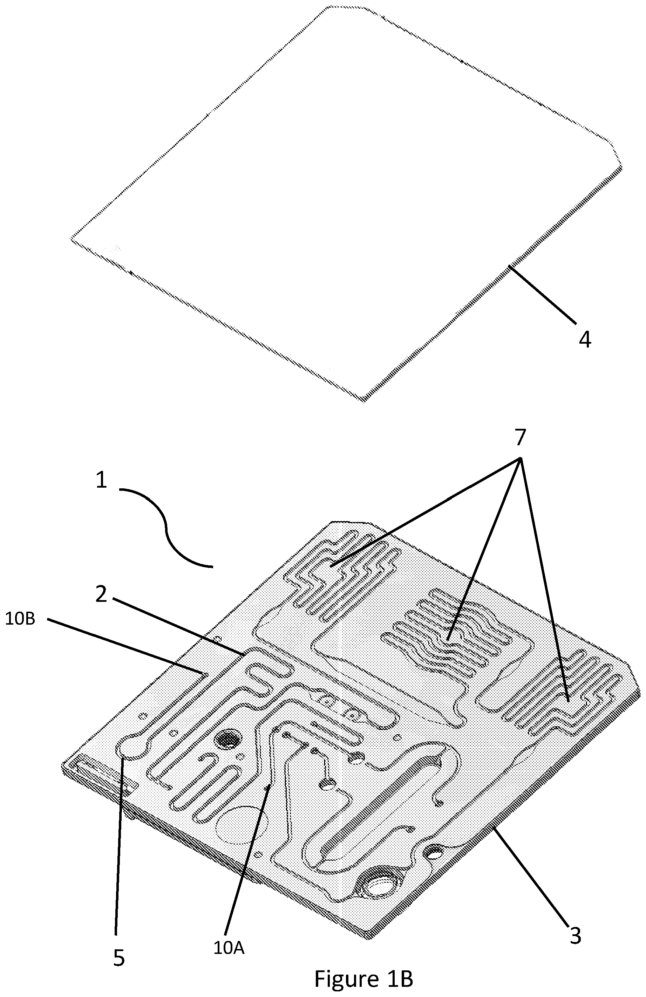

Figure 1a provides a perspective view of a microfluidics cassette according to an aspect of the present invention, the view showing an outer surface; Figure 1b provides an exploded perspective view of a similar microfluidics cassette shown from the other side, with various internal features visible.

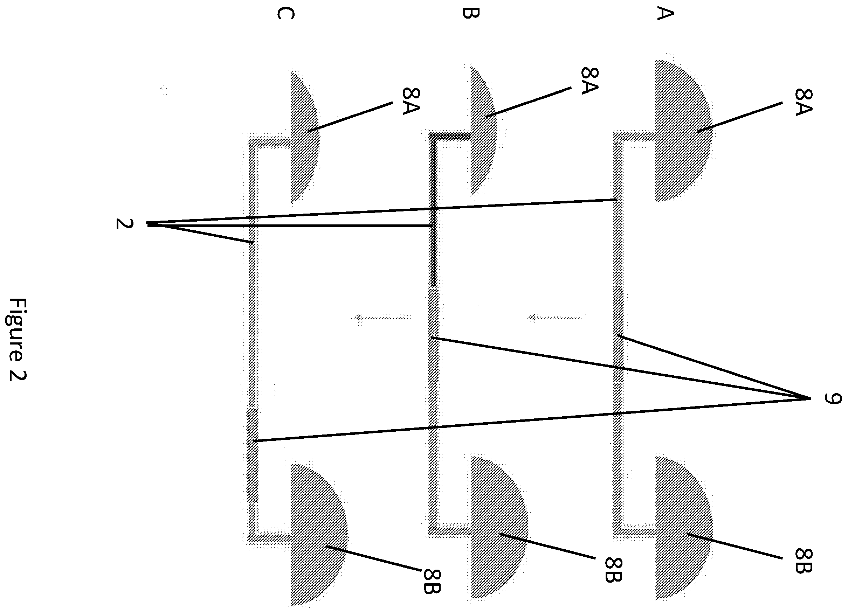

Figure 2a, 2b, 2c is a simplified diagram showing the use of two bellows pumps to pressurise a sealed area of a cassette, with the first applying pressure and the second acting as a reservoir; and

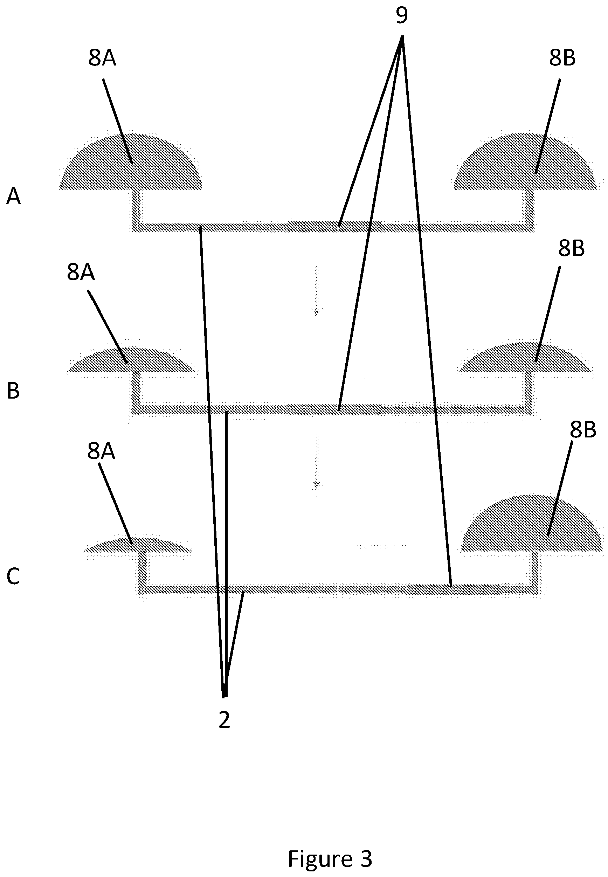

Figure 3a, 3b, 3c is a simplified diagram showing the use of two bellows pumps that are actuated simultaneously pressurise a sealed area of a cassette. Fig 3B shows the pressurization step while Fig. 3C shows the reciprocation mode to move the sample while keeping the pressure in a sellable portion of the cassette.

### Detailed Description

According to a first aspect of the present invention, and as generally depicted in figure 1, there is provided a microfluidic cassette 1, more generally termed a 'device', with a micro-channel 2, where the micro-channel 2 allows for continuous flow-through of fluid as required. As shown most clearly in Figure 1b, which shows the internal workings of a similarly configured cassette, the micro-channel 2 is formed inside the microfluidic cassette 1, in the desired length and shape to allow the passage of a sample, typically a biological sample in liquid format, and/or reagents, some of which may be incorporated on-cassette during the flow-through, along a fluid flow path and through various zones or areas which allow different activities to occur including amplification of DNA from the sample by polymerase chain reaction (PCR). A PCR section of the channel 7 is a portion of the channel that has been configured to allow thermocycling of a sample passing therethrough to occur. The PCR section 7 of the channel (and cassette) is specifically adapted to allow a liquid sample travelling therethrough to be heated and cooled in a cyclical manner to temperatures that allow for denaturing of DNA, annealing of DNA and simplification of DNA. The PCR section 7 of the channel is heatable either by an integral heat source within the channel or the wall of the device, or more typically by being brought into the proximity of an external heat source which applies heat to microfluidic device (such as a microfluidic cassette) in a manner that allows heat to transfer into the PCR section of the channel.

Various valves and fluidically intercommunicating offshoots such as additional channels, reservoirs or chambers can be used to allow mixing, washing, removal and other actions to occur according to the needs of a diagnostic or biochemical assay to be performed therein in an automated or semi-automated manner. The assay is generally carried out by allowing a sample to interact and/or react with one or more reagents in one or more steps; typically in one or more channels or chambers of the device, for times and at temperatures effective in forming a detectable product that indicates the presence or absence of an analyte in the sample. The channel 2 is formed in a first surface of a first substrate 3, typically a substantially planar, substantially rigid substrate which in this embodiment is polypropylene. The first substrate 3 is overlaid with a second substrate 4, which in this embodiment is a polypropylene film. By bonding the first substrate material 3 to the film 4, for example using laser welding, a substantially closed channel 2 is provided (inlets and outlets can be included as required). It will be understood that if the first substrate 3 is a planar element with an upper and lower surface, the majority of the microchannel 2 can formed in the upper surface or the lower surface. It is however often desirable that the second substrate, i.e. the film 4, forms the upper wall of the microchannel 2 in use. The use of laser welding ensured that a fluid-tight, and more specifically an air-tight seal is created around the microchannels, such that no air can escape or leak out between the first and second substrates. It will be understood that methods other than laser welding can be used and will typically be selected based on the substrate materials themselves. The seal or weld is sufficiently strong to withstand increased pressure levels within the cassette compare to outside of the cassette. Typically, it is strong enough to withstand internal gauge pressures of at least 200 kPa (2bar).

Alternatively, it would be understood that although this embodiment has the second substrate as a film 4, the second substrate can be another material and may itself have grooves or channel formed on its surface that can be aligned with the channels of the first substrate. By bonding the substrates 3, 4 together, a closed channel 2 is provided (again inlets and outlets can be included as required).

The cartridges are typically consumables; i.e., they are used once and then discarded; and contain all or many of the reagents needed for one or more assays to be performed. Such reagents can be held on the cartridge in reservoirs or similar.

Where necessary, the first and second substrates 3, 4 can be aligned prior to bonding. The length and cross-sectional shape of the channel 2 can be any appropriate shape to allow for the desired transport and processing of a sample and or reagents. Such microfluidic systems 'lab-on-a-chip' type systems being well known in the art.

The cassette 1 is provided with an inlet 5 for receiving a sample into the microfluidic channel. The inlet 5 is provided with an air-tight seal in the form of an insertable cap 6. The insertable cap 6 is hinged to the surface of the cassette for ease of use. The cap 6 is sized to be received into the aperture of the inlet and is provided with a resiliently deformable collar that acts to form an air-tight seal in the inlet after the sample has been inserted and when the cap 6 is closed.

In this embodiment, the other opposite end of the microchannel 2 to the inlet 5 is permanently sealed as it is a single use cassette. However, a similar cap to that used on the inlet could be used to close an outlet if present. In an alternative embodiment, valves positioned within the microchannel, away from the inlet, can be used to create a smaller volume air-tight area if required (still containing the PCR amplification zone, or PCR section 7). For example, a first air-tight valve could be provided proximate and upstream of the amplification zone 7 and a second air-tight valve could be provided proximate and upstream of the amplification zone 7. Once the sample is between the two valves these can be closed and the pressure in the microchannel 2, containing the amplification zone 7, which is between the two valves raised as in other embodiments.

The microchannel 2 is a continuous flow channel and, in this embodiment, it includes an amplification zone or PCR section 7 which includes three portions of channel that are heatable to different temperatures to allow the thermocycling of temperatures. In this embodiment there is a portion of the channel in the PCR section 7 that is heated to a temperature of 90 degrees to 98 degrees C. There is another portion of the channel in the PCR section 7 that is heated to a temperature of around 45 degrees to 70 degrees C. A further portion of the channel in the PCR section 7 is heated to a temperature of around 70 degrees to 75 degrees C. In this embodiment the heating of the channel occurs when an external heater source is brought into close proximity with the cassette 1 such that heat exchange occurs through the wall of the cassette into the relevant portions of the micro-channel. The microchannel in the amplification zone or PCR section 7 is serpentine in shape such that the sample will travel through the different temperature zones as it travels along each section of the serpentine shape. In this embodiment the heaters are provided in a separate host instrument which receives the cassette therein, but in theory the heaters could form part of the cassette, for example being positioned within the channels themselves. There are also temperature sensors positioned within or close to the relevant pats of the channel to allow the temperature in the channel to be read and if necessary controlled. Although in this embodiment a serpentine shaped microchannel 2 is used in the PCR section 7 to ensure sample moving through the channel is cyclically taken through the different temperatures required for a PCR reaction to occur, it would be understood that other layouts can be used to thermocycle a sample through different temperatures in a microfluidic cassette. For example, a shuttle flow system could be used in place of the serpentine shaping. In a shuttle flow variant, the sample is shuttled back and forward between two or more sections of the microchannel (as opposed to continuously moving forward as in the serpentine variant), each section being held at one of the required temperatures for a PCR reaction to occur.

The microchannel includes a first bellows pump 8A in fluid communication with the microchannel 2 (via bellows inlet 10A). The first bellows pump 8A is a positive displacement pump, comprising a hemispherical, compressible bellow which is resiliently biased to return to its expanded shape. The internal dimensions of the hemispherical bellow can be changed by applying pressure to its external surface. c. In the present embodiment, mechanical actuators found on a host instrument into which the cassette is placed act under the control of a microprocessor to actuate the bellows pumps by compression, partial compression, partial decompression or decompression of the same. The actuators could however, in theory, be incorporated onto the cassette.

The microchannel also includes a second bellows pump **8B** in fluid communication with the microchannel 2 (via bellows inlet 10B). The second bellows pump 8B is also a positive displacement pump, in this example again comprising a hemispherical, compressible bellow which is resiliently biased to return to its expanded shape. The second bellows 8B can be partially compressed or decompressed as well as fully compressed or decompressed.

The microchannel 2 also includes various other reservoirs, branches and chambers (such as a capture and viewing chamber for example) as are required to carry out a molecular assay. Where present, the host instrument will include elements such as actuators, heaters and optical elements.

Whilst this example describes bellows pumps with a hemispherical bellow, it would be understood by those skilled in the art that other bellows formations could be used, or indeed alternative types of positive displacement pump could be used. For example, another type of positive displacement pump is a syringe pump in which the displacement of the piston of a syringe inside a syringe cylinder causes fluid to be sucked into or pressed out of the syringe outlet.

In use, a liquid sample **9** is introduced into the inlet 5, which leads to the micro-fluidic channel 2 and the cap 6 is closed to form an air-tight seal. The cassette 1 is placed into a host instrument (not shown). In a first embodiment, as depicted in a basic form in figure 2, at rest, air pressure on either side of the liquid sample 9 is equal (see fig 2A). The hemispherical bellow 8A is then actuated by pins present in the host instrument pressing down on its external surface and thus reducing the volume that air can fill between the bellow and the liquid sample. The host instrument pins or actuators can be provided with sensors to detect the surface of the bellows to ensure specific control of the pressure is achieved. As the cassette is now a sealed (air-tight) system the air has nowhere to go, so the same amount of air is in a smaller volume, so the pressure of the air must increase (see fig 2B where there is an increase in pressure to the left of the sample which will act to push the sample 9 to the right). As the air pressure behind the liquid sample 9 is higher than the air pressure in front of the liquid sample, the liquid sample is pushed forward until the air pressure before and after balance again (see Fig 2C - both sides have equal pressure again). In this embodiment the second bellow 8B is acting as a pressure 'reservoir' thus dampening some of the movement of the sample as the relative change in pressure is not as large, meaning the sample can still move, but pressure inside the channel can stay under 200 kPa (2 bar) (but still increase compared to air pressure outside of the sealed cassette). In this embodiment, the absolute pressure inside the channel is increased to 160 kPa (1.6bar) prior to the sample being amplified in the amplification zone.

Notably, if only one bellow is used, the pressure would still be applied, but the movement of the sample would be much greater. This excessive movement and pressure can be avoided (i.e. dampened) even with only one bellow by using much longer channels, but this requires a greater footprint on the cassette which is typically undesirable.

Whilst the above example describes how the second bellow can be used as a reservoir, figure 3 is a preferred variation of the present invention which shows that utilising a plurality of positive displacement pumps such as bellows or diaphragm pumps has additional benefits. The preferred use of the present invention is to include at least two bellows pumps (or other positive displacement pumps) to induce an increase in pressure to at least 160 kPa (1.6 bar) within the microchannel 2. Again, in use, a liquid sample **9** is introduced into the inlet 5, and into the micro-fluidic channel 2; and the cap 6 is closed to form an air-tight seal. The cassette 1 is placed into a host instrument (not shown). Prior to the cassette being closed by the cap 6, both of bellows pump 8A and bellows pump 8B are fully decompressed. In the two examples above, the volume of the entire system changes whenever bellow 8A is depressed. This requires constantly changing the pressure inside the cassette during the assay itself which makes fluid control within the microchannel more difficult. Therefore, in this preferred embodiment, in order to increase the pressure in the sealed portion of the microchannel 2 which contains the amplification zone or PCR section 7, both first bellow 8A and second bellow 8B are actuated simultaneously to keep the overall volume of the system equal, and so keep the overall pressure of the system equal. The host instrument applies a push force to the external surfaces of both first bellow 8A and second bellow 8B such that they are compressed by a first amount (the bellows are compressed approximately half-way) to increase the pressure inside the cassette to approx. 1.6 bar. This can be seen in Fig 3B and it can be seen that the sample at this point does not move if bellows pump 8A is positioned on the opposite side of the sample to bellows pump 8B i.e. a bellow is provided at either side of the sample, (in this case either side of the inlet 5 into which the sample was initially inserted) and in fact preferably at either side of the amplification zone. At this stage the bellows pumps 8A and 8B can continue to be used to move the liquid sample 9 within the channel as required by the assay. By including various valves, and actuating them to open and close as required, this can allow sample to be very effectively moved throughout various regions of the micro-channel such that different actions and reactions can be carried out. In order to move the liquid sample 9 to the right, bellows pump 8A can be further compressed by a second amount and Bellow B allowed to re-inflate (the resilient material of the bellows allowing it to revert back to its original hemispherical shape). Using this mechanism of concurrently or simultaneously actuating or compressing the two valves - or using two positive displacement pumps simultaneously to raise the pressure in the portion of the microchannel containing the amplification zone 7 prior to the liquid sample 9 being amplified (which requires the heating and cooling to different temperatures) means the pressure inside always stays at 160 kPa (1.6bar) and the liquid sample 9 can also be moved around using the same positive displacement pumps without the danger of increasing the pressure within the microchannel to a point where the bonds, such as laser bonds, valves or cap will leak.

It is preferred that the pressure in the portion of the microchannel containing the amplification zone is increased to 160 kPa (1.6 bar) prior to thermocycling occurring in the amplification zone to obtain the benefits of reducing bubble formation, as well as ensuring that the system works even at high altitude, whilst not damaging or exceeding the limits of any of the valves, caps or seals present in the cassette. However, the pressure could also be raised to between 150 kPa (1.5bar) and 200 kPa (2bar) and significant beneficial effects would still be seen. In some embodiments even raising the pressure to 110 kPa (1.1bar) or 120 kPa (1.2bar) or higher would still allow the benefits of the system working at higher altitudes and some reduction in bubble formation when compared to a standard system where no pressure increase prior to amplification would typically occur.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations).

## Claims

1. A microfluidic device comprising;
a microfluidic fluidic channel having a polymerase chain reaction (PCR) section (7), said PCR section configured to allow the temperature changes required for polymerase chain reaction (PCR) to occur therein,
a fluidically isolatable portion of the device (2) comprising at least the portion of the fluidic channel having a PCR section,
at least one closure means actuatable to fluidically isolate the fluidically isolatable portion from the external environment, and
a first bellows pump (8A) and a second bellows pump (8B), and;
wherein, the first bellows pump and the second bellows pump are arranged such that in use they are simultaneously or concurrently compressed by a first amount to a first position to increase the pressure in the isolatable portion to a raised pressure, and then the first pump is further compressed by a second amount and the second pump allowed to reinflate to move a liquid sample within the channel without further increasing the pressure in the isolatable portion beyond the raised pressure.

2. A microfluidic device according to any of the previous claims wherein isolatable portion is the entire fluidic channel present on the device.

3. A microfluidic device according to any of the previous claims wherein the device comprises at least one air-tight valve or sealing means actuatable to fluidically isolate the fluidically isolatable portion.

4. A diagnostic system comprising the microfluidic device of any of the previous claims and a host instrument able to receive said microfluidic device.

5. A diagnostic system as in Claim 4 wherein the host instrument comprises an interface enabled to convey pressure from said host instrument to said bellows pumps on said microfluidic device.

6. A diagnostic system as in any of Claims 4 to 5 wherein the host instrument comprises a microprocessor which controls the interactions between the host instrument and the microfluidic device.

7. A method of amplifying nucleic acid from a sample comprising,
providing the device of any of Claims 1 to 6,
introducing a sample into the microfluidic device,
fluidically isolating at least a portion of the device comprising at least the PCR section, increasing the pressure within the isolated portion,
and, after the pressure has been increased within the isolated portion, carrying out polymerase chain reaction (PCR) amplification steps to amplify nucleic acid within the sample,
wherein the step of increasing the pressure within the isolated portion comprises simultaneously compressing the first bellows pump and the second bellows pump by a first amount to increase the pressure in the isolated portion to a raised pressure, and the first bellow pump is then further compressed by a second amount and the second pump allowed to reinflate to move the sample within the channel without further increasing the pressure in the isolatable portion beyond the raised pressure.

8. A method of amplifying nucleic acid as in Claim 7 wherein one or more fluid-tight valves are closed to fluidically isolate the entire device or only a portion of the microfluidic channel containing the PCR section is fluidically isolated.

9. A method of amplifying nucleic acid as in any of Claims 7 to 8 wherein the step of increasing the pressure to a raised pressure increases the pressure in the isolated portion to between 120kPa (1.2bar) and 200kPa (2bar).

10. A microfluidic device as in any of Claims 1 to 5 or a method of amplifying nucleic acid as in any of Claims 7 to 9, wherein the raised pressure is between 150kPa (1.5bar) and 200kPa (2bar).

11. A microfluidic device as in any of Claims 1 to 5 or a method of amplifying nucleic acid as in any of Claims 7 to 9, wherein the raised pressure is 160kPa (1.6bar).

## Patentansprüche

1. Mikrofluidische Vorrichtung, umfassend:
einen mikrofluidischen Fluidikkanal, der einen Abschnitt (7) für die Polymerasekettenreaktion (PCR) aufweist, wobei der PCR-Abschnitt konfiguriert ist, um die für die Polymerasekettenreaktion (PCR) erforderlichen Temperaturänderungen darin auftreten zu lassen,
einen fluidisch isolierbaren Abschnitt der Vorrichtung (2), der mindestens den Abschnitt des Fluidikkanals umfasst, der einen PCR-Abschnitt aufweist,
mindestens ein Verschlussmittel, das betätigt werden kann, um den fluidisch isolierbaren Abschnitt von der äußeren Umgebung zu isolieren, und
eine erste Balgpumpe (8A) und eine zweite Balgpumpe (8B), und;
wobei die erste Balgpumpe und die zweite Balgpumpe so angeordnet sind, dass sie im Gebrauch gleichzeitig oder gleichzeitig um einen ersten Betrag in eine erste Position komprimiert werden, um den Druck in dem isolierbaren Abschnitt auf einen erhöhten Druck zu erhöhen, und dann die erste Pumpe weiter um einen zweiten Betrag komprimiert wird und die zweite Pumpe wieder aufblasen kann, um eine flüssige Probe innerhalb des Kanals zu bewegen, ohne den Druck in dem isolierbaren Abschnitt über den erhöhten Druck hinaus weiter zu erhöhen.

2. Mikrofluidische Vorrichtung nach einem der vorstehenden Ansprüche, wobei der isolierbare Abschnitt der gesamte auf der Vorrichtung vorhandene Fluidikkanal ist.

3. Mikrofluidische Vorrichtung nach einem der vorstehenden Ansprüche, wobei die Vorrichtung mindestens ein luftdichtes Ventil oder eine Dichtung umfasst, das/die betätigt werden kann, um den fluidisch isolierbaren Abschnitt fluidisch zu isolieren.

4. Diagnostisches System, umfassend die mikrofluidische Vorrichtung nach einem der vorstehenden Ansprüche und ein Host-Instrument, das die mikrofluidische Vorrichtung empfangen kann.

5. Diagnostisches System nach Anspruch 4, wobei das Host-Instrument eine Schnittstelle umfasst, die es ermöglicht, Druck von dem Host-Instrument zu den Balgpumpen auf der mikrofluidischen Vorrichtung zu übertragen.

6. Diagnostisches System nach einem der Ansprüche 4 bis 5, wobei das Host-Instrument einen Mikroprozessor umfasst, der die Interaktionen zwischen dem Host-Instrument und der mikrofluidischen Vorrichtung steuert.

7. Verfahren zum Amplifizieren von Nukleinsäure aus einer Probe, umfassend:
Bereitstellen der Vorrichtung nach einem der Ansprüche 1 bis 6,
Einführen einer Probe in die mikrofluidische Vorrichtung, fluidisches Isolieren mindestens eines Abschnitts der Vorrichtung, der mindestens den PCR-Abschnitt umfasst, Erhöhen des Drucks innerhalb des isolierten Abschnitts, und, nachdem der Druck innerhalb des isolierten Abschnitts erhöht wurde, Durchführen von Amplifikationsschritten der Polymerasekettenreaktion (PCR), um Nukleinsäure innerhalb der Probe zu amplifizieren,
wobei der Schritt des Erhöhens des Drucks innerhalb des isolierten Abschnitts das gleichzeitige Zusammendrücken der ersten Balgpumpe und der zweiten Balgpumpe um einen ersten Betrag umfasst, um den Druck in dem isolierten Abschnitt auf einen erhöhten Druck zu erhöhen, und die erste Balgpumpe dann um einen zweiten Betrag weiter zusammengedrückt wird und die zweite Pumpe wieder aufpumpen darf, um die Probe innerhalb des Kanals zu bewegen, ohne den Druck in dem isolierbaren Abschnitt über den erhöhten Druck hinaus weiter zu erhöhen.

8. Verfahren zum Amplifizieren von Nukleinsäure nach Anspruch 7, wobei ein oder mehrere fluiddichte Ventile geschlossen werden, um die gesamte Vorrichtung fluidisch zu isolieren, oder nur ein Abschnitt des mikrofluidischen Kanals, der den PCR-Abschnitt enthält, fluidisch isoliert wird.

9. Verfahren zum Amplifizieren von Nukleinsäure nach einem der Ansprüche 7 bis 8, wobei der Schritt des Erhöhens des Drucks auf einen erhabenen Druck den Druck in dem isolierten Abschnitt auf zwischen 120 kPa (1,2 bar) und 200 kPa (2 bar) erhöht.

10. Mikrofluidische Vorrichtung nach einem der Ansprüche 1 bis 5 oder ein Verfahren zum Amplifizieren von Nukleinsäure nach einem der Ansprüche 7 bis 9, wobei der erhöhte Druck zwischen 150 kPa (1,5 bar) und 200 kPa (2 bar) liegt.

11. Mikrofluidische Vorrichtung nach einem der Ansprüche 1 bis 5 oder ein Verfahren zum Amplifizieren von Nukleinsäure nach einem der Ansprüche 7 bis 9, wobei der erhöhte Druck 160 kPa (1,6 bar) beträgt.

## Revendications

1. Dispositif microfluidique comprenant ;
un canal fluidique microfluidique ayant une section de réaction en chaîne par polymérase (PCR) (7), ladite section PCR étant conçue pour permettre les changements de température nécessaires à la réaction en chaîne par polymérase (PCR) qui se produit à l'intérieur de celle-ci,
une partie fluidiquement isolable du dispositif (2) comprenant au moins la partie du canal fluidique ayant une section PCR,
au moins un moyen de fermeture actionnable pour isoler fluidiquement la partie isolable fluidiquement de l'environnement extérieur, et
une première pompe à soufflet (8A) et une seconde pompe à soufflet (8B), et ;
dans lequel la première pompe à soufflet et la seconde pompe à soufflet sont agencées de telle sorte qu'en utilisation, elles sont comprimées simultanément ou concurremment d'une première quantité jusqu'à une première position afin d'augmenter la pression dans la partie isolable jusqu'à une pression élevée, et ensuite la première pompe est comprimée davantage d'une seconde quantité et la seconde pompe est autorisée à se regonfler pour déplacer un échantillon liquide à l'intérieur du canal sans augmenter davantage la pression dans la partie isolable au-delà de la pression élevée.

2. Dispositif microfluidique selon l'une quelconque des revendications précédentes, dans lequel la partie isolable est l'ensemble du canal fluidique présent sur le dispositif.

3. Dispositif microfluidique selon l'une quelconque des revendications précédentes, dans lequel le dispositif comprend au moins une valve étanche à l'air ou un moyen d'étanchéité pouvant être actionné pour isoler fluidiquement la partie isolable fluidiquement.

4. Système de diagnostic comprenant le dispositif microfluidique selon l'une quelconque des revendications précédentes et un instrument hôte capable de recevoir ledit dispositif microfluidique.

5. Système de diagnostic selon la revendication 4, dans lequel l'instrument hôte comprend une interface permettant d'acheminer la pression dudit instrument hôte vers lesdites pompes à soufflet sur ledit dispositif microfluidique.

6. Système de diagnostic selon l'une quelconque des revendications 4 à 5, dans lequel l'instrument hôte comprend un microprocesseur qui contrôle les interactions entre l'instrument hôte et le dispositif microfluidique.

7. Procédé d'amplification d'acide nucléique à partir d'un échantillon, comprenant,
la fourniture du dispositif selon l'une quelconque des revendications 1 à 6,
l'introduction d'un échantillon dans le dispositif microfluidique,
l'isolation de manière fluidique d'au moins une partie du dispositif comprenant au moins la section PCR, ce qui augmente la pression à l'intérieur de la partie isolée, et, après que la pression a été augmentée à l'intérieur de la partie isolée, l'exécution des étapes d'amplification par réaction en chaîne par polymérase (PCR) pour amplifier l'acide nucléique à l'intérieur de l'échantillon,
dans lequel l'étape d'augmentation de la pression à l'intérieur de la partie isolée comprend la compression de manière simultanée de la première pompe à soufflet et de la seconde pompe à soufflet d'une première quantité pour augmenter la pression dans la partie isolée jusqu'à une pression élevée, et la première pompe à soufflet est ensuite comprimée davantage d'une seconde quantité et la seconde pompe est autorisée à se regonfler pour déplacer l'échantillon à l'intérieur du canal sans augmenter davantage la pression dans la partie isolable au-delà de la pression élevée.

8. Procédé d'amplification d'acide nucléique selon la revendication 7, dans lequel une ou plusieurs vannes étanches aux fluides sont fermées afin d'isoler fluidiquement l'ensemble du dispositif, ou seule une partie du canal microfluidique contenant la section PCR est isolée fluidiquement.

9. Procédé d'amplification d'acide nucléique selon l'une quelconque des revendications 7 à 8, dans lequel l'étape d'augmentation de la pression à une pression élevée augmente la pression dans la partie isolée entre 120 kPa (1,2 bar) et 200 kPa (2 bar).

10. Dispositif microfluidique selon l'une quelconque des revendications 1 à 5 ou procédé d'amplification d'acide nucléique selon l'une quelconque des revendications 7 à 9, dans lequel la pression élevée est comprise entre 150 kPa (1,5 bar) et 200 kPa (2 bar).

11. Dispositif microfluidique selon l'une quelconque des revendications 1 à 5 ou procédé d'amplification d'acide nucléique selon l'une quelconque des revendications 7 à 9, dans lequel la pression élevée est de 160 kPa (1,6 bar).
